# EUROPÄISCHE PATENTANMELDUNG

(11) **EP 1 238 682 A2**
(43) Veröffentlichungstag der Anmeldung: **11.09.2002**
(21) Anmeldenummer: 02004983.9
(22) Anmeldetag: 06.03.2002
(51) Int. Cl.: A61M 39/02

(54) **Port für ein implantierbares Port-System**

(30) Priorität: 08.03.2001 DE 20103979 U
(71) Anmelder: CareMed Medical Produkte Aktiengesellschaft, 01109 Dresden (DE)
(72) Erfinder: Reuter, Jürgen, 36211 Alheim (DE); Kreibich, Hans-Jürgen, 61350 Bad Homburg (DE); Wex, Roland, 34212 Melsungen (DE)
(74) Vertreter: Pätzelt, Peter, Dipl.-Ing.

(57) **Zusammenfassung**

Die Erfindung betrifft einen Port für ein implantierbares Port-System, welches aus mindestens einem Port mit einem Gehäuse (1) und einem Katheteranschluss (8) sowie einem Katheter besteht. Am Gehäuse (1) des Ports ist beidseitig und parallel zur Achse des Katheteranschlusses (8) je mindestens eine konkave Ausnehmung (9, 10) vorhanden. In einer Ausgestaltung sind die konkaven Ausnehmungen (9, 10) an einer Grundplatte (2) am Gehäuse (1) ausgebildet.

## Beschreibung

Die Erfindung betrifft einen Port für ein implantierbares Port-System, welches mindestens aus einem Port mit einem Gehäuse und einem Katheteranschluss sowie einem Katheter besteht.

Nach dem Stand der Technik sind verschiedenartige Port-Systeme bekannt. Dabei ist die äußere Kontur der Gehäuse der Ports meist rund ausgebildet. Oft ist eine Grundplatte vorhanden, die das eigentliche Gehäuse radial überragt. Soweit zwei oder mehr Ports parallel angeordnet sind, entsteht ein ovales oder langgestrecktes Gehäuse.

Im Inneren der Ports befinden sich Portkammern oder Reservoirs, in die über ein Septum ein Medikament eingebracht werden kann. Die Zuführung der Medikamente in eine Vene erfolgt über den Katheter. Der Anschluss des Katheters an die Portkammer erfolgt üblicherweise über einen Rohrstutzen am Gehäuse, der mit der Portkammer verbunden ist. Der Katheter wird bei der Implantation auf den Rohrstutzen aufgesteckt.

Dieses Aufstecken erfolgt durch den Chirurgen, der dabei Handschuhe trägt. Die Handschuhe sind bei der Implantation zwangsweise feucht und der Chirurg hat einige Mühe, den Port zu halten und den Katheter sicher aufzustecken.

Der Erfindung liegt damit als Aufgabe zugrunde, einen Port der eingangs genannten Art anzugeben, der mit geringem technischen Aufwand die Handhabbarkeit des Ports bei der Konnektion und Implantation für den Chirurgen erleichtert.

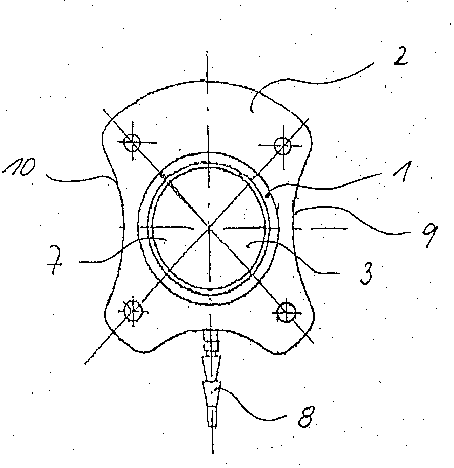

Die Erfindung löst die Aufgabe durch die im kennzeichnenden Teil des Anspruchs 1 angegebenen Merkmale. Vorteilhafte Weiterbildungen der Erfindung sind in den Unteransprüchen gekennzeichnet und werden nachstehend zusammen mit der Beschreibung der bevorzugten Ausführung der Erfindung, einschließlich der Zeichnung, näher dargestellt.

Erfindungsgemäß sind am Port beidseitig und parallel zur Achse des Katheteranschlusses je mindestens eine konkave Ausnehmung vorhanden. Dabei gehören zur Erfindung auch solche Ausnehmungen, die im geometrischen Sinn nicht konkav gewölbt sind, sondern in anderer Weise eine Ausnehmung darstellen, in die der Chirurg mit den Fingern eingreifen kann, derart, dass Kräfte in der Achse des Katheteranschlusses mit den Fingern sicher beherrscht werden.

Vorteilhaft ist die Ausbildung nach Anspruch 2, bei der die konkaven Ausnehmungen an einer Grundplatte am Gehäuse ausgebildet sind.

Bei zwei oder mehr Ports, die zu einem Block zusammengefasst sind, ist es vorteilhaft die konkaven Ausnehmungen an den zur Achse der Katheteranschlüsse parallelen äußeren Seiten auszubilden.

Der Vorteil des erfindungsgemäßen Ports besteht darin, dass der Port bei der Implantation und insbesondere beim Aufstecken des Katheters sicher gehandhabt werden kann.

Die Erfindung wird nachstehend an einem Ausführungsbeispiel näher erläutert. Die zugehörige Zeichnung zeigt einen erfindungsgemäßen Port in der Draufsicht.

Der Port besteht aus einem Gehäuse 1 und einer unteren Grundplatte 2. Innerhalb des Gehäuses ist eine Portkammer 3 ausgebildet, die als Reservoir für das jeweilige Medikament dient. Oben ist die Portkammer 3 mit einem Septum abgedeckt.

Zum Anschluss eines in der Zeichnung nicht dargestellten Katheters führt ein rohrförmiger Auslass von der Portkammer 3 durch die Grundplatte 2 zu einem Katheteranschluss 8, der in Form eines bekannten Schlauchanschlussstutzens ausgebildet ist. Der Auslass mit einem Katheteranschluss kann auch gesondert parallel zur Grundplatte ausgebildet sein.

Die eigentliche Erfindung besteht in der Ausbildung der konkaven Ausnehmungen 9 und 10 am äußeren Rand der Grundplatte 2. Diese konkaven Ausnehmungen 9 und 10 befinden sich beidseitig des Gehäuses 1, also im Wesentlichen gegenüberliegend, und parallel zur Achse des Katheteranschlusses 8.

Bei der Implantation des Ports bzw. des gesamten Portsystems kann der Chirurg den Port sehr vorteilhaft zwischen Daumen und Zeigefinger problemlos sicher fassen und den Katheter auf den Katheteranschluss 8 aufschieben.

Die Erfindung ist selbstverständlich nicht auf das beschriebene Ausführungsbeispiel beschränkt. So ist es ohne weiteres möglich, die konkaven Ausnehmungen 9 und 10 durch äquivalente Ausnehmungen in fachmännischer Weise an gegebene Ports oder Mehrfach-Ports anzupassen.

## Patentansprüche

1. Port für ein implantierbares Port-System, welches aus mindestens einem Port mit einem Gehäuse (1) und einem Katheteranschluss (8) sowie einem Katheter besteht, **dadurch gekennzeichnet, dass** am Gehäuse (1) des Ports beidseitig und parallel zur Achse des Katheteranschlusses (8) je mindestens eine konkave Ausnehmung (9, 10) vorhanden ist.

2. Port nach Anspruch 1, **dadurch gekennzeichnet, dass** die konkaven Ausnehmungen (9, 10) an einer Grundplatte (2) am Gehäuse (1) ausgebildet sind.

3. Port nach Anspruch 1 oder 2, **dadurch gekennzeichnet, dass** bei Mehrfach-Ports die konkaven Ausnehmungen an den zur Achse des Katheteranschlusses parallelen äußere Seiten ausgebildet sind.
